# EUROPÄISCHE PATENTANMELDUNG

(11) **EP 2 056 226 A1**
(43) Veröffentlichungstag der Anmeldung: **06.05.2009**
(21) Anmeldenummer: 09152683.0
(22) Anmeldetag: 21.12.2004
(51) Int. Cl.: G06F 19/00, A61B 5/00, A23L 1/00, A23L 1/29, A23L 1/30, A61B 5/083, A61B 5/107

(54) **Diätetisches Lebensmittel**

(30) Priorität: 16.01.2004 EP 04000848
(62) Teilanmeldung aus: 04030252.3
(71) Anmelder: PM-International AG, 1618 Luxembourg (LU)
(72) Erfinder: Dr. Schmitt, Gerhard, 64625, Bensheim (DE)
(74) Vertreter: Zech, Stefan Markus

(57) **Zusammenfassung**

Die Erfindung betrifft ein diätetisches Lebensmittel, beinhaltend wenigstens ein Sojaeiweiß, wenigstens einen Mikrofaser-Ballaststoff, wenigstens einen Kohlenhydrat-Lieferanten, wenigstens einen Fettsäure-Lieferanten, wenigstens ein fettlösliches und wenigstens ein wasserlösliches Vitamin, wenigstens einen Mineralstoff, wenigstens einen Geschmacksstoff und Isoflavone.

## Beschreibung

Die Erfindung betrifft ein diätetisches Lebensmittel.

Übergewicht und Bewegungsmangel sind häufige Ursachen für chronische Erkrankungen wie Bluthochdruck, Herzerkrankungen oder Gelenkarthrose. Um diesen Erkrankungen vorzubeugen oder die Heilung zu unterstützen ist daher bei übergewichtigen Personen eine kontrollierte Gewichtsabnahme empfehlenswert. Eine Gewichtsreduktion kann zudem erwünscht sein, um ein persönliches Wohlfühlgewicht oder eine schönere Figur zu erreichen.

In der Regel wird unter Übergewicht Fettübergewicht verstanden, das überwiegend auf zu viele Kalorien, zu fettreiche und zu süße Nahrung zurückzuführen ist, aber auch auf unregelmäßiges, einseitiges und zu spätes Essen. Eine besonders ungünstige Kombination ist dabei Zucker mit Fett wie in Fastfood mit Cola, Kuchen oder Schokoladen usw. enthalten. Über die Ausschüttung des Hormons Insulin wird massiv Fett gespeichert und gleichzeitig über diese ungünstige Kombination von Zucker und Fett (von der Überzuckerung in die Unterzuckerung) der nächste Heißhunger provoziert. Durch unregelmäßiges Essen mit vielen kleinen, süßen Zwischenmahlzeiten wird die Fettverbrennung zusätzlich blockiert. Zucker wiederum fördert die Übersäuerung und damit auch die zweite Art des Übergewichts. Fettübergewicht ist nur schwer wieder abzubauen und es bedarf eines längeren Zeitraums.

Ganz anders verhält es sich beim Wasser-Schlackenübergewicht. Durch eine permanente Übersäuerung des Körpers (über 90% der Bevölkerung sind übersäuert), hervorgerufen durch einseitige Ernährung (Fleisch, Alkohol, Kaffee etc.), falsche Bewegung, Stress, falsche Atmung usw., sind die Neutralisations- und Ausscheidungssysteme (Niere, Lunge) für Säuren erschöpft. Der Körper versucht die Säuren über Mineralien (z.B. aus den Knochen) abzupuffern und lagert sie in Kombination mit Wasser ein, vielfach in neu gebildeten Fettzellen. Wasser dient auch dazu Säuren zu verdünnen. Es entwickelt sich ein "Ganzkörperödem" das Übergewicht und Cellulite zur Folge hat. Wasser-Schlackenübergewicht ist durch eine gründliche Entsäuerung relativ rasch und schnell abzunehmen.

Bekannte Methoden zur Reduzierung des Körpergewichts bzw. des Körperfettanteils sind Bewegungstraining sowie eine kalorienarme Ernährung. Aus DE 100 23 141 A1 beispielsweise ist ein Verfahren zur Durchführung einer zeitoptimierten Ernährungsanalyse für einen beliebigen Probanden zur Erfassung seiner Nähr- und Wirkstoffversorgung, beispielsweise als Grundlage für ein Beratungsgespräch, bekannt, bei dem zur Bewertung des Verhältnisses von Energiezufuhr und Energieverbrauch die über einen vorgegeben Zeitraum aufgenommene feste und/oder flüssige Nahrung sowie der in diesem Zeitraum durch körperliche Aktivitäten bedingten Energieverbrauch mit Hilfe eines Computers mit einer speziell angepassten Software unter Berücksichtigung der Körperstruktur (Körperzusammensetzung in Bezug auf die Muskelmasse) des Probanden ausgewertet und das Auswerteergebnis auf einem Drucker ausgegeben wird. Auf Grundlage dieser Daten können dann beispielsweise individuelle Diätpläne für den Probanden ausgearbeitet und Empfehlungen für körperliche Aktivitäten gegeben werden. Auch können Defizite im Essverhalten ermittelt und durch ergänzende Mittel wie Nahrungsergänzung, Diätprodukte oder Arzneimittel kompensiert werden. Die Einhaltung eines Diätplans erfordert jedoch ständige Kontrolle und anhaltende Konsequenz. Die Erfolgsquote solch einer Verfahrensweise ist daher in der Praxis relativ gering.

Ein Verfahren zur Gewichtsregulierung, bei dem die Diät stetig überwacht und dem aktuellen Diäterfolg angepasst wird, ist in DE 43 34 149 A1 beschrieben. In einem elektronischen Datenerfassungs- und Auswertegerät wird bei dem Verfahren aus den persönlichen Daten des Anwenders ein Optimalgewicht errechnet, mit dem aktuellen Gewicht verglichen und daraus der optimale Kalorienbedarf ermittelt und mit Wertetabellen von Lebensmitteln verglichen. Hierbei wird der optimale Kalorienbedarf pro Tag berechnet und nach einem Zeitraum von mehr als drei Tagen ein neues aktuelles Gewicht bestimmt. Die über diesen Zeitraum eingenommen Lebensmittel werden nach Art und Menge erfasst und daraus der durchschnittliche, tägliche Kalorienaufnahmewert (IstWert) ermittelt, mit dem errechneten optimalen Kalorienbedarf (Soll-Wert) verglichen und ein neuer optimaler Kalorienbedarf pro Tag errechnet. Dieses Verfahren ist jedoch einseitig, da es auf die Überwachung der täglichen Kalorienzufuhr beschränkt ist. Zudem wird vom Anwender eine anhaltende Konsequenz in der Auswahl der Lebensmittel und Zubereitung der Speisen gefordert, um in einem vernünftigen Zeitraum zu einem Diäterfolg zu gelangen.

Aus der Adipositas-Behandlung ist eine Vorrichtung (DE 198 19 257 C2) mit einem Körperübungsgerät und einem separaten PC bekannt, wobei an den PC ein Ergometer zur Bestimmung der mit dem Körperübungsgerät geleisteten Arbeit, eine Waage, ein Bioimpedanz-Messgerät und ein Pulsfrequenz-Messgerät angeschlossen ist, wobei an den PC vom Ergometer Signale zur Leistungsbestimmung übertragen werden, mit dem PC der Body-Mass-Index und anhand des Bioimpedanz-Messgeräts die Körperzusammensetzung des Patienten ermittelt wird sowie die Trainingsdaten vom PC gespeichert und über den gesamten Trainingszeitraum verglichen werden können. Werden die vorgegebenen Therapiemaßnahmen nicht eingehalten, gibt eine Anzeige ein negatives Feed-back, bei Einhaltung der therapeutischen Empfehlungen wird ein positives Feed-back gegeben. Diese Vorrichtung zielt auf den langzeitigen Abbau von Fettübergewicht durch eine Umstellung der Lebensgewohnheiten der Patienten. Es erfolgt jedoch keine Anpassung des Trainingsprogramms auf den aktuellen Trainingserfolg. Die Vorrichtung ist daher nicht für die direkte Steuerung eines Diätverlaufs geeignet.

Es ist nun Aufgabe der Erfindung, ein diätetisches Lebensmittel bereitzustellen, das den gezielten Abbau von Fettübergewicht und Wasser-Schlackenübergewicht ermöglicht und insbesondere flexibel auf eine Vielzahl von Diätprogrammen Anwendung finden kann.

Diese Aufgabe wird durch ein diätetisches Lebensmittel gemäß Anspruch 1 gelöst. Das diätetische Lebensmittel beinhaltet wenigstens ein Sojaeiweiß, wenigstens einen Mikrofaser-Ballaststoff, wenigstens einen Kohlenhydrat-Lieferanten, wenigstens einen Fettsäure-Lieferanten, wenigstens ein fettlösliches Vitamin und wenigstens ein wasserlösliches Vitamin, wenigstens einen Mineralstoff, wenigstens einen Geschmacksstoff sowie Isoflavone.

Das diätetische Lebensmittel ist somit speziell für eine wirksame Gewichtsabnahme durch kalorienreduzierte Mahlzeiten mit Unterstützung der Thermogenese (Verbrennung von Kalorien in Wärme) aber auch für eine sichere und gesunde Gewichtsabnahme durch die Ausgewogenheit der wertvollen und lebenswichtigen Nähr- und Vitalstoffe konzipiert. Das diätetische Lebensmittel wird bevorzugt als Shake hergestellt.

Hochwertiges pflanzliches Sojaeiweiß mit einer sehr hohen biologischen Wertigkeit hilft die stoffwechselaktive und formgebende Muskulatur zu erhalten und damit die Gewichtsabnahme dauerhaft zu sichern. Durch den ausschließlichen Einsatz von Sojaeiweiß ist das diätetische Lebensmittel zudem frei von Milcheiweiß. Weiterhin ist das Lebensmittel lactosefrei. Dadurch ist es auch für entsprechende Allergiker verträglich.

Die Mikrofaser-Ballaststoffe bremsen den Heißhunger über einen verlangsamten Zuckereinstrom ins Blut und sorgen zudem für eine Entgiftung und eine bessere Verdauung. Die Kohlenhydrate garantieren Leistungsfähigkeit während der Gewichtsabnahme auch für das Gehirn und das Nervensystem. Darüber hinaus liefert das diätetische Lebensmittel lebensnotwendige Fettsäuren, Vitamine und Mineralstoffe. Mit Hilfe der Geschmacksstoffe werden verschiedene Geschmacksrichtungen erzeugt, so dass eine Abwechslung bei der Einnahme des diätetischen Lebensmittels entstehen kann und darüber hinaus für jede Person die richtige Geschmacksrichtung angeboten werden kann.

Isoflavone sind sekundäre Pflanzenstoffe und wirken als Phytohormone (Phytoöstrogene). Durch den Zusatz von Isoflavonen können unter anderem Wechseljahrsbeschwerden verringert, gesunde stabile Knochen gefördert sowie Osteoporose und Brustkrebs vorgebeugt werden. Darüber hinaus können Isoflavone im Zusammenhang mit dem Cholesterinspiegel den LDL-Wert erniedrigen und den HDL-Wert erhöhen sowie das Prostata-Krebs-Risiko beim Mann reduzieren. Isoflavone wirken Hormonspiegel normalisierend und dienen als Herz-Kreislauf-Schutz.

Das diätetische Lebensmittel greift speziell beim Fettübergewicht an, das längerfristig mobilisiert werden muss. Es wird empfohlen, ein bis zwei Mahlzeiten täglich durch das diätetische Lebensmittel zu ersetzen.

Insbesondere vorteilhaft ist es, wenn der Isoflavongehalt zwischen 10 mg/100g diätetisches Lebensmittel und 200 mg/100g diätetisches Lebensmittel, vorzugsweise zwischen 30 mg/100g diätetisches Lebensmittel und 60 mg/100g diätetisches Lebensmittel, insbesondere bei 33 mg/100g diätetisches Lebensmittel, liegt.

Darüber hinaus kann es vorteilhaft sein, wenn das wenigstens eine fettlösliche Vitamin, insbesondere Vitamin A, D und E, in micellierter Form vorliegt. Auf diese Weise kann das Vitamin dem menschlichen Organismus besser verfügbar gemacht werden.

Da das Transportsystems des menschlichen Körpers auf "Wasserbasis" funktioniert, haben es fettlösliche Substanzen schwer bei der Aufnahme aus dem Darm in die Lymphe bzw. das Blut. Der Körper baut im Normalfall mit Hilfe von Gallensäuren, Glyceriden und Fettsäuren sogenannte Micellen auf, in die fettlösliche Substanzen wie manche Vitamine für die Resorption eingeschlossen werden. Die Resorptionsrate von fettlöslichen Vitaminen aus der Nahrung ist deshalb relativ gering (nur 25-30% bei Vitamin E und Provitamin A). Der Körper schafft es, in der Zeit, in der der Speisebrei den Zwölffingerdarm passiert, ca. 25% der fettlöslichen Vitamine in Micellen zu "verpacken" und damit resorbierbar zu machen. Werden die Vitamine bereits in micellierter Form zugeführt, kann in derselben Zeit ein wesentlich größerer Anteil im Körper resorbiert werden.

Vorzugsweise ist das diätetische Lebensmittel zur Verwendung in einem Verfahren zur Überwachung bzw. Steuerung eines Diäterfolgs während einer Diät vorgesehen, die unter Einnahme wenigstens eines Diäthilfsmittels in einer vorgegebenen Dosierung erfolgt und durch ein vorgegebenes Bewegungsprogramm und/oder ein vorgegebenes Ernährungsprogramm unterstützt wird, werden zunächst ein oder mehrere anfängliche Körperwerte W₀ ermittelt und dann in vorgegebenen oder beliebigen Zeitabständen zur Erzielung aktualisierter Vorgabewerte Vₐₖₜ für die Dosierung des wenigstens eines Diäthilfsmittels, eines vorgegebenen Bewegungsprogramms und/oder eines vorgegebenes Ernährungsprogramms wenigstens einmal erneut ermittelt, unter Berücksichtigung des wenigstens einen aktuellen Körperwertes Wₐₖₜ sowie ggf. unter Berücksichtigung zuvor ermittelter Körperwerte W_{akt-Z} (z= 1 .. akt), wobei zumindest die Dosierung des wenigstens einen Diäthilfsmittels eingestellt wird, bis der mindestens eine aktuelle Körperwert Wₐₖₜ in einem als Diäterfolg definierten Zielbereich liegt,
wobei
k >= 0,
1 >= 0,
Wₓ = [W_{0,x},.. , W_{k,x}],
Vₓ = [V_{0,x},.. , V_{1,x}],
und k bzw. 1 die Anzahl unterschiedlicher Körperwerte bzw. Vorgabe-
werte zu einem Zeitpunkt tₓ ist.

Als Diäterfolg ist grundsätzlich eine Gewichtsreduktion anzusehen, wobei insbesondere auch der Körperfettanteil erniedrigt wird. Es kann aber auch als Diäterfolg gewertet werden, wenn bei gleichbleibendem Gewicht eine Erniedrigung des Körperfettanteils oder eines Körpermaßes erreicht wird. Zu Beginn der Diät wird vorzugsweise ein Sollwert für einen Körperwert als Maß für einen endgültigen Diäterfolg vorgegeben, beispielsweise ein bestimmtes Körpergewicht, ein bestimmter Körperfettanteil oder bestimmte Körpermaße. Sollte es sich als vorteilhaft erweisen, kann dieser Sollwert während des Diätverlaufs verändert, insbesondere angepasst oder korrigiert, werden.

Der Körperwert ist ein repräsentativer Wert, über den sich der Diäterfolg bzw. der Diätverlauf sicher kontrollieren lässt. Besonders vorteilhaft kann es sein, wenn in den vorgegebenen oder beliebigen Zeitabständen gleichzeitig mehrere Körperwerte bestimmt werden, so dass eine sehr genaue Überwachung des Diäterfolgs möglich ist und die Dosierung des Diäthilfsmittels genau angepasst werden kann.

Die vorgegebenen oder beliebigen Zeitabstände können Tage, Wochen oder Monate umfassen und mit der Fortdauer der Diät unterschiedlich lang sein. Beispielsweise kann es zweckmäßig sein zu Beginn der Diät in kurzen Zeitabständen, beispielsweise ein bis mehrere Tage, den wenigstens einen Körperwert Wₐₖₜ zu ermitteln, da zu Beginn mit einer relativ raschen Gewichtsreduktion zu rechnen ist. Im weiteren Verlauf der Diät können die Zeitabstände für die Ermittlung des wenigstens einen aktuellen Körperwerts Wₐₖₜ dann verlängert werden, beispielsweise auf Wochen- oder Monatsrhythmus, da nach den ersten Diäterfolgen eine weitere Gewichtsabnahme bzw. Reduzierung des Körperfettanteils relativ langsam vonstatten geht.

Vorzugsweise ist das diätetische Lebensmittel Bestandteil eines Systems bzw. einer Anordnung zur Überwachung bzw. Steuerung eines Diäterfolgs, während einer Diät, die unter Einnahme wenigstens eines Diäthilfsmittels in einer vorgegebenen Dosierung erfolgt und durch ein vorgegebenes Bewegungsprogramm und/oder ein vorgegebenes Ernährungsprogramm unterstützt wird, insbesondere zur Durchführung des oben geschilderten Verfahrens. Das System bzw. die Anordnung umfasst wenigstens eine Einrichtung zur Ermittlung wenigstens eines aktuellen Körperwerts Wₐₖₜ, eine vorgegebene Menge wenigstens eines Diäthilfsmittels und wenigstens ein Datenverarbeitungsprogramm zur Auswertung des wenigstens einen aktuellen Körperwerts Wₐₖₜ, wobei mit Hilfe des Datenverarbeitungsprogramms in Abhängigkeit des ermittelten wenigstens einen aktuellen Körperwerts Wₐₖₜ zumindest eine optimale Dosierung des wenigstens einen Diäthilfsmittels bestimmbar ist.

Das Datenverarbeitungsprogramm ist vorzugsweise so gestaltet, dass es mit einer beliebigen Datenverarbeitungseinheit, beispielsweise ein PC, ein Laptop, ein tragbarer Kleinrechner (PDA - Personal Digital Assistant) oder ein Großrechner, angewendet werden kann, und einfach in der Bedienung. Über die Auswertung des wenigstens einen aktuellen Körperwerts Wₐₖₜ, ggf. unter Berücksichtigung zuvor ermittelter Körperwerte W_{akt-z} (z=1..akt), kann der Fortschritt der Diät bzw. der Diäterfolg beobachtet bzw. überwacht werden. Diäterfolg und ermittelte Vorgabewerte Vₐₖₜ einer aktuellen Dosierung des wenigstens einen Diäthilfsmittels sowie weitere diätspezifische Daten werden von dem Datenverarbeitungsprogramm vorzugsweise als Grafik und/oder Tabelle ausgegeben bzw. ausgelesen.

Durch regelmäßige Kontrolle eines oder mehrerer aktueller Körperwerte Wₐₖₜ, ggf. unter Berücksichtigung zuvor ermittelter Körperwerte W_{akt-z} (z= 1 .. akt), kann der aktuelle Diäterfolg ermittelt werden, um sofort auf diesen reagieren zu können und zumindest die Dosierung eines oder mehrerer Diäthilfsmittel entsprechend einzustellen bzw. anzupassen, das bedeutet, je nach aktuellem Diäterfolg bzw. Diätverlauf zu erhöhen oder zu erniedrigen. Im Extremfall kann im Diätverlauf das Diäthilfsmittel auch ganz weggelassen werden, beispielweise wenn ersichtlich ist, dass sich der gewünschte, endgültige Diäterfolg allein durch das unterstützende Bewegungsprogramm und/oder Ernährungsprogramm einstellt. Auf diese Weise kann eine für den Körper schonende, gut steuerbare Diät mit langanhaltendem Ergebnis erzielt werden.

Vorzugsweise werden die einen oder mehreren aktuellen Körperwerte über mehrere Zeitpunkte aktualisiert, mindestens über zwei oder drei verschiedene ausreichend auseinanderliegende Ermittlungszeitpunkte, um so nicht nur Informationen über den tatsächlichen Ist-Zustand der Körperwerte zu erhalten, sondern auch unter Berücksichtigung der zur Anwendung gelangten Diät-Hilfsmittel den vorherigen Verlauf der Diät, d.h. das Reaktionsvermögen des Patienten auf die Diät-Hilfsmittel erfassen zu können.

Weiterhin kann die Diät individuell auf den Menschentyp abgestimmt werden. Das kann beispielsweise durch einen Vergleich eines oder mehrerer aktueller Körperwerte Wₐₖₜ mit zuvor ermittelten Körperwerten W_{akt-z} (z=1..akt) erfolgen. Hieraus lässt sich beispielsweise ermitteln, wie die Person auf eine vorgegebene Kombination von wenigstens einem Diäthilfsmittel und/oder Bewegungsprogramm und/oder Ernährungsprogramm reagiert. Auf diese Weise lässt sich ein für diese Person optimales Programm bestimmen.

Weiterhin vorteilhaft ist, dass mit dem Verfahren gemäß den Merkmalen des Anspruchs 4 die Abnahme des Fettübergewichts und des Wasser-Schlackenübergewichts gefördert und dabei die Muskulatur als stoffwechselaktives Gewebe bestmöglich erhalten wird. Über das Diäthilfsmittel werden dem Körper beispielweise wichtige Nährstoffe, Vitamine und Mineralstoffe oder auch Stoffe, die die Gewichtsreduzierung fördern, zugeführt. Somit kann die Diät in relativ kurzer Zeit und ohne zu "Hungern" durchgeführt werden.

In einer besonders bevorzugten Ausführungsform des Verfahrens, werden bzw. wird in Abhängigkeit des ermittelten wenigstens einen aktuellen Körperwerts Wₐₖₜ, ggf. unter Berücksichtigung zuvor ermittelter Körperwerte W_{akt-z} (z= 1
.. akt), das vorgegebene Bewegungsprogramm und/oder das vorgegebene Ernährungsprogramm angepasst bis der gewünschte endgültige Diäterfolg erreicht ist. Durch die zusätzliche Anpassung des Bewegungsprogramms lässt sich insbesondere gezielt erreichen, dass bei der Diät nicht nur das Gewicht, sondern auch der Körperfettanteil reduziert und die Muskelmasse verbessert wird. Das führt zum einen zu einer Straffung des Körpers und zum anderen zu einem erhöhten Energiegrundumsatz im Körper und damit zu einem langanhaltenden Diäterfolg. Das Bewegungsprogramm umfasst vorzugsweise eine tägliche sportliche Ausdaueraktivität in einem Zielpulsbereich für die Fettverbrennung. Als sportliche Aktivitäten eignen sich besonders Laufen, schnelles Gehen (Walking), im besonderen mit Unterstützung von Stöcken (Nordic Walking), Schwimmen oder Rad fahren, sowie weitere Ausdaueraktivitäten unter Zuhilfenahme von Fitnessgeräten.

Durch die vom Diäterfolg bzw. von dem wenigstens einen aktuellen Körperwert Wₐₖₜ, ggf. unter Berücksichtigung zuvor ermittelter Körperwerte W_{akt-z} (z= 1 .. akt), abhängige Anpassung des Ernährungsprogramms kann die jeweilige Person insbesondere bei einem positiven Diäterfolg langsam zu einer normalen Ernährung (erhöhte Kalorienzufuhr) zurückgeführt werden, die einen anhaltenden Diäterfolg sicherstellt. Bei negativem Diäterfolg wird das Ernährungsprogramm ebenfalls entsprechend angepasst und die Kalorienzufuhr verringert. Das Ernährungsprogramm kann beispielsweise einen Diätplan mit genauer Angabe der Kalorienzufuhr bzw. der zu verzehrenden Lebensmittel und Speisen umfassen. Das Bewegungsprogramm und das Ernährungsprogramm können zu Beginn der Diät mit einem beliebigen bekannten Verfahren erstellt werden.

Insbesondere vorteilhaft kann es sein, wenn in Abhängigkeit des ermittelten wenigstens einen aktuellen Körperwerts Wₐₖₜ, ggf. unter Berücksichtigung zuvor ermittelter Körperwerte W_{akt-z} (z= 1 .. akt), wenigstens ein Diäthilfsmittel ausgetauscht und/oder in Abhängigkeit des ermittelten wenigstens einen aktuellen Körperwerts Wₐₖₜ, ggf. unter Berücksichtigung zuvor ermittelter Körperwerte W_{akt-z} (z= 1 .. akt), wenigstens ein weiteres Diäthilfsmittel abgesetzt wird. So kann es beispielsweise vorteilhaft sein zu Beginn der Diät ein Diäthilfsmittel zur Entsäuerung des Körpers oder allgemein zur Förderung der Gewichtsabnahme einzunehmen und dieses dann ab einem bestimmten Zeitpunkt abzusetzen, während beispielsweise ein Diäthilfsmittel als Mahlzeitenersatz oder ein Nahrungsergänzungsmittel über den gesamten Zeitraum der Diät in jeweils angepasster Dosierung eingenommen werden.

Bei dem Verfahren werden bzw. wird bevorzugt als Körperwert(e) der Body-Mass-Index und/oder das Körpergewicht und/oder die Körperzusammensetzung, insbesondere der Körperfettgehalt und/oder der Körperwassergehalt, und/oder wenigstens ein Körpermaß, insbesondere der Hüftumfang und/oder der Taillenumfang und/oder der Brustumfang und/oder der Oberschenkelumfang und/oder das Taille/Hüfte-Verhältnis, und/oder ein Maß für die körperliche Leistungsfähigkeit, insbesondere der PWC-Wert und/oder der Puls, ermittelt.

Besonders vorteilhaft kann es sein, wenn in den vorgegebenen Zeitabständen gleichzeitig mehrere aktuelle Körperwerte Wₐₖₜ bestimmt werden. Allein die Bestimmung des Gewichts beispielsweise gibt keinen eindeutigen Befund über den Diäterfolg, denn ein gleichbleibendes Gewicht bei steigender Muskelmasse und reduzierter Fettmasse ist bereits ein Erfolg. Durch die Erhöhung der Muskelmasse steigt der Energiegrundumsatz des Menschen und somit verbessert sich die Fähigkeit sowohl Gewicht zu reduzieren als auch das Gewicht später zu halten.

Der Body-Mass-Index (BMI) lässt sich aus der Körpergröße und dem Gewicht nach der Gleichung Gewicht (kg) geteilt durch das Quadrat der Körpergröße (m) bestimmen. Ein BMI im Bereich von 20 - 25 wird als ideal angesehen und sollte daher angestrebt werden. Bei einem BMI von < 20 wird der Bereich des Untergewichts erreicht. Ein BMI zwischen 25 und 30 zeigt leichtes Übergewicht, ein BMI > 31 starkes Übergewicht an.

Für eine Körperfettanalyse können alle bekannten Verfahren eingesetzt werden, darunter die Unterwassermethode, die NIR (Near-Infrarot)-Methode oder ein Impedanzmessgerät. Am einfachsten in der Handhabung sind die Impedanzmessgeräte, beispielsweise Tanita Fettwaagen oder Omron Handmessgeräte. Der bei dieser Methode gemessene Körperwiderstand ist abhängig vom Körperwasseranteil, das Messgerät misst also nicht den Fettanteil, sondern den Körperwasseranteil. Damit ist die Methode Schwankungen unterworfen, zum Beispiel durch Wasser- oder Nahrungsaufnahme, Medikamente, sportliche Aktivitäten, etc., und es ist sehr genau darauf zu achten, dass stets unter vergleichbaren Bedingungen gemessen wird. Bei Schwangeren oder Personen mit Herzschrittmacher sollte jedoch nicht nach der Impedanzmethode gemessen werden.

Für den Körperfettanteil gelten die in Tabelle 1 und Tabelle 2 angeführten Richtwerte. Als Diäterfolg werden in der Regel Werte im Bereich "Gesund" angesehen bzw. angestrebt.

**Tabelle 1: Körperfettanteil bei erwachsenen Frauen**

| Alter | erhöhtes Risiko | Gesund | erhöhtes Risiko | hohes Risiko |
|---|---|---|---|---|
| | %Fett | %Fett | %Fett | %Fett |
| 20-39 | <21 | 21-32 | 33-38 | >38 |
| 40-59 | <23 | 23-33 | 34-39 | >39 |
| 60-79 | <24 | 24-35 | 36-41 | >41 |

**Tabelle 2: Körperfettanteil bei erwachsenen Männern**

| Alter | erhöhtes Risiko | Gesund | erhöhtes Risiko | hohes Risiko |
|---|---|---|---|---|
| | %Fett | %Fett | %Fett | %Fett |
| 20-39 | <8 | 8-19 | 20-24 | >24 |
| 40-59 | <11 | 11-21 | 22-27 | >27 |
| 60-79 | <13 | 14-24 | 25-29 | >29 |

Körpermaße können mit einer entsprechenden Messeinrichtung, beispielsweise mit einem Maßband, gemessen werden. Für die Bestimmung, des Taille/Hüfte-Verhältnisses (WHR = Waist to Hip Ratio), ein Indikator für die Körperfettverteilung, wird der Taillenumfang (W) direkt unterhalb des Rippenbogens und der Hüftumfang (H) an der breitesten Stelle des Hüftknochens gemessen. Angestrebt wird ein W/H-Verhältnis von < 0,85 (gynoide Form, Birnenform). Bei einem W/H-Verhältnis > 1,0 (androide Form, Apfelform) besteht nach wissenschaftlichen Erkenntnissen ein deutlich höheres Gesundheitsrisiko.

Der PWC-Wert (Physical-Work Capacity ) ist ein Index für die körperliche Leistungsfähigkeit eines Patienten bei einer bestimmten Herzfrequenz. Für die PWC wurden empirisch ermittelte Werte für Männer und Frauen in den verschiedenen Altersstufen als Sollwerte aufgestellt. Diese PWC-Tabellen können der Literatur entnommen werden, wobei die Tabellen verschiedener Autoren bezüglich der Leistungshöhe in geringem Maße differieren.

Besonders vorteilhaft ist es, wenn der ermittelte wenigstens eine aktuelle Körperwert Wₐₖₜ und/oder wenigstens eine dem Körperwert Wₐₖₜ zugeordnete Zeitangabe tₐₖₜ, insbesondere ein Datum oder eine Zeitspanne zu einem vorhergehend ermittelten wenigstens einen aktuellen Körperwert W_{akt-z} (z= 1 .. akt), und/oder wenigstens ein Personenparameter, insbesondere die Größe und/oder das Geschlecht und/oder das Alter und/oder wenigstens ein körperlicher Zustand und/oder wenigstens eine medizinische Information, an eine Datenverarbeitungseinheit übertragen wird oder in eine Datenverarbeitungseinheit eingegeben wird und die Datenverarbeitungseinheit die Dosierung des wenigstens einen Diäthilfsmittels und/oder das Bewegungsprogramm und/oder das Ernährungsprogramm in Abhängigkeit des wenigstens einen aktuellen Körperwerts Wₐₖₜ, ggf. unter Berücksichtigung zuvor ermittelter Körperwerte W_{akt-z} (z= 1 .. akt), und/oder der wenigstens einen dem aktuellen Körperwert Wₐₖₜ zugeordneten Zeitangabe tₐₖₜ und/oder des wenigstens einen Personenparameters ermittelt. Die Datenverarbeitungseinheit kann als herkömmlicher PC, Laptop, tragbarer Kleinrechner (PDA - Personal Digital Assistant) oder als Großrechner ausgeführt sein. Die Körperwerte, Zeitangaben und Personenparameter können der Datenverarbeitungseinheit über eine Verbindung zur Signalübertragung zur Verfügung gestellt, beispielsweise über ein internes Netzwerk oder eine Internetverbindung, oder von Hand in die Datenverarbeitungseinheit eingegeben werden.

Als Personenparameter bzw. Personenwert werden charakteristische Werte und Eigenschaften einer Person bezeichnet, die zur Aufstellung eines Diätplans oder während des Diätverlaufs berücksichtigt werden sollten, von der Diät selbst jedoch in der Regel nicht unbedingt beeinflusst werden. Zu den Personenparametern zählen insbesondere Werte wie das Geschlecht, das Alter oder die Größe der jeweiligen Person sowie spezielle Eigenschaften oder körperliche Zustände wie Raucher/Nichtraucher, Schwangerschaft und Stillzeit. Die Personenparameter können darüber hinaus medizinische Informationen umfassen, zum Beispiel chronische Krankheiten wie Diabetes, Herz- /Kreislauferkrankungen, Allergien oder Osteoporose sowie Angaben über ständig einzunehmende Arzneimittel.

Vorzugsweise vergleicht dann in der Datenverarbeitungseinheit ein Datenverarbeitungsprogramm den wenigstens einen aktuellen Körperwert Wₐₖₜ, ggf. unter Berücksichtigung zuvor ermittelter Körperwerte W_{akt-z} (z= 1 .. akt), (Istwert) mit einem optimalen Körperwert (Sollwert) und/oder einem zu einem vorgehenden Zeitpunkt ermittelten Körperwert und ermittelt das Datenverarbeitungsprogramm die Dosierung des wenigstens einen Diäthilfsmittels und/oder das Bewegungsprogramm und/oder das Ernährungsprogramm in Abhängigkeit des wenigstens einen aktuellen Körperwerts Wₐₖₜ, ggf. unter Berücksichtigung zuvor ermittelter Körperwerte W_{akt-z} (z=1..akt), und/oder der wenigstens einen dem aktuellen Körperwert Wₐₖₜ zuordnete Zeitangabe tₐₖₜ und/oder des wenigstens einen Personenparameters.

Die Zeitabstände, in denen der wenigstens eine ermittelte aktuelle Körperwert Wₐₖₜ dem Datenverarbeitungsprogramm zur Verfügung gestellt wird, können vom Datenverarbeitungsprogramm vorgegeben sein. Bevorzugt ist es jedoch, wenn ein ermittelter aktueller Körperwert Wₐₖₜ und eine zugehörige Zeitangabe tₐₖₜ, insbesondere ein Datum, oder eine Zeitspanne zu einem vorhergehend ermittelten wenigstens einen Körperwert W_{akt-z} (z=1..akt), an die Datenverarbeitungseinheit übertragen werden oder in die Datenverarbeitungseinheit eingegeben werden und das Datenverarbeitungsprogramm die Dosierung des wenigstens einen Diäthilfsmittels und/oder das Bewegungsprogramm und/oder das Ernährungsprogramm in Abhängigkeit des wenigstens einen aktuellen Körperwerts Wₐₖₜ und der wenigstens einen Zeitangabe tₐₖₜ ermittelt.

Das ermöglicht eine genaue Überwachung bzw. Steuerung des Diäterfolgs, auch dann wenn der wenigstens eine aktuelle Körperwert Wₐₖₜ in einem beliebigen Zeitabstand gemessen wird. Das Datenverarbeitungsprogramm ermittelt dann beispielsweise einen in dieser Zeitspanne oder bis zu diesem Zeitpunkt theoretisch zu erreichenden Körperwert (Sollwert), vergleicht diesen mit dem tatsächlich ermittelten wenigstens einen aktuellen Körperwert Wₐₖₜ, ggf. unter Berücksichtigung zuvor ermittelter Körperwerte W_{akt-z}, (z=1..akt), (Istwert) und ermittelt auf Grundlage dieser Daten eine entsprechende Dosierung des wenigstens einen Diäthilfsmittels und/oder ein Bewegungsprogramm und/oder ein Ernährungsprogramm, die bzw. das zu dem gewünschten, endgültigen Diäterfolg führen können bzw. kann.

Besonders bevorzugt ist es zudem, wenn der Diäterfolg, insbesondere der wenigstens eine aktuelle Körperwert Wₐₖₜ, und/oder die Dosierung des wenigstens einen Diäthilfsmittels und/oder das Bewegungsprogramm und/oder das Ernährungsprogramm als Grafik und/oder als Tabelle von dem Datenverarbeitungsprogramm ausgegeben und/oder aus dem Datenverarbeitungsprogramm ausgelesen werden kann.

In einer besonders vorteilhaften Weiterbildung des Verfahrens werden Daten mehrerer Personen, insbesondere der jeweilige wenigstens eine aktuelle Körperwert Wₐₖₜ und/oder die wenigstens eine dem aktuellen Körperwert Wₐₖₜ zugeordnete Zeitangabe tₐₖₜ und/oder der wenigstens eine Personenparameter, in eine zentrale Datenverarbeitungseinheit eingegeben oder an die zentrale Datenverarbeitungseinheit übertragen, insbesondere über eine Datenverbindung, vorzugsweise über das Internet, ermittelt das Datenverarbeitungsprogramm personenspezifisch die Dosierung des wenigstens einen Diäthilfsmittels und/oder das Bewegungsprogramm und/oder das Ernährungsprogramm, die bzw. das an die jeweilige Person ausgegeben oder ausgelesen und übertragen werden bzw. wird.

Die zentrale Datenverarbeitungseinheit kann beispielsweise in einem Unternehmen, einer Organisation, einer Praxis oder in einer Klinik eingerichtet sein. Dort wird dann für die jeweilige Person ein persönlicher Diätplan zusammengestellt. Zur Überwachung des Diäterfolgs kann dann die Person in vorgegebenen Zeitabständen die zentrale Stelle aufsuchen, in der ihre Körperwerte ermittelt werden und auf Basis dieser Körperwerte ein angepasster Diätplan erstellt wird. Eine weitere Möglichkeit zur Überwachung des Diätplans besteht darin, dass die jeweilige Person ihre Körperwerte in den vorgegebenen Zeitabständen, beispielsweise über den Postweg, das Telefonnetz oder eine sonstige Datenverbindung, an die zentrale Stelle bzw. Datenverarbeitungseinheit übermittelt und auf demselben Weg wiederum einen angepassten Diätplan erhält. Spezielle Geräte oder Einrichtungen zur Bestimmung der Körperwerte können von der zentralen Stelle gegebenenfalls leihweise zur Verfügung gestellt werden. Auf diese Weise kann der Diäterfolg stets sicher überwacht werden.

In einer besonders bevorzugten Ausführungsform des Verfahrens wird als Diäthilfsmittel wenigstens ein Mittel zur Entsäuerung, insbesondere ein basischer Tee und/oder ein Basenpulver, und/oder ein diätetisches Lebensmittel als Mahlzeitenersatz und/oder ein Nahrungsergänzungsmittel und/oder ein Wirkstoffpräparat eingenommen.

Als Mittel zur Entsäuerung kann ein Basenpulver, beispielsweise das Produkt Restorate^{®}, eingenommen werden. Das Basenpulver unterstützt durch die Entsäuerung den Abbau des Fettübergewichts, da es die Fettzellen aufschließt, und fördert gleichzeitig über die Entsäuerung den Abbau des Wasser-Schlackenübergewichts. Eine Gewichtsabnahme durch Entsäuerung findet relativ schnell statt und kann bei "verschlackten" Personen über Wasser-Schlackenausscheidung rasch zu hohen Gewichtsabnahmen führen. Eine ausreichende Entsäuerung verhindert auch dauerhaft die erneute Einlagerung von Wasser und Schlacken und unterstützt den Fettabbau. Je nach Übersäuerung werden ein bis drei Rationen Basenpulver pro Tag empfohlen.

Als Mittel zur Entsäuerung kann weiterhin ein basischer Tee Anwendung finden, beispielweise das Produkt Herbaslim^{®}. Der basische Tee liefert bei der Gewichtsabnahme wichtige Flüssigkeit zur Neutralisation und zum Abtransport der freigesetzten Schlacken und Gifte aus den Fettzellen. Ausreichendes Trinken (mind. 1 1 des basischen Tees plus viel Wasser ohne Kohlensäure) verhindert damit auch auf einfache Weise mögliche "Fastennebenwirkungen" wie Kopfschmerzen oder Mundgeruch. Der basische Tee unterstützt, auch in Kombination mit dem Basenpulver, die Entsäuerung und damit den Abbau des Wasser-Schlackenübergewichts.

Diätetische Lebensmittel dienen einem besonderen Ernährungszweck, beispielsweise bei Krankheiten, bei Mangelerscheinungen (z.B. Vitaminmangel), oder bei Übergewicht. Sie werden nur in Fertigpackungen angeboten und sind mit Angaben über die ernährungsbezogenen Eigenschaften versehen. Als diätetisches Lebensmittel kann beispielweise ein aufzulösendes Pulver, ein Brei, ein Shake oder eine Flüssigkeit zugeführt werden, die in seiner Zusammensetzung speziell für eine wirksame, sichere und gesunde Gewichtsabnahme entwickelt wurden. Es enthält dann alle Inhaltsstoffe einer kalorienreduzierten Mahlzeit. Das diätetische Lebensmittel greift speziell beim Fettübergewicht an, das längerfristig mobilisiert werden muss. Es wird empfohlen ein bis zwei Mahlzeiten täglich durch das diätetische Lebensmittel zu ersetzen.

Die besten Diäterfolge können erzielt werden wenn wenigstens über einen beschränkten Zeitraum die vorgenannten Mittel zur Entsäuerung und das diätetische Lebensmittel in Kombination eingenommen werden. Darüber hinaus können Nahrungsergänzungsmittel wie Vitamin- oder Mineralstoffpräparate oder Wirkstoffpräparate, die Wirkstoffe zur Gewichtsreduzierung enthalten, die Diät zusätzlich unterstützen.

Bei dem System bzw. der Anordnung zur Überwachung bzw. Steuerung eines Diäterfolgs ist es besonders bevorzugt, wenn mit Hilfe des Datenverarbeitungsprogramms in Abhängigkeit des ermittelten wenigstens einen aktuellen Körperwerts Wₐₖₜ, ggf. unter Berücksichtigung zuvor ermittelter Körperwerte W_{akt-z} (z= 1 .. akt), das Bewegungsprogramm und/oder das vorgegebene Ernährungsprogramm und/oder die Art des wenigstens einen Diäthilfsmittels und/oder die Art und/oder die Anzahl weiterer Diäthilfsmittel bestimmbar ist sowie in Abhängigkeit eines Diätverlaufs eine Anpassung oder Abänderung der Vorgabewerte Vₐₖₜ für die Dosierung des wenigstens einen Diäthilfsmittels und/oder des Bewegungsprogramms und/oder des vorgegebenen Ernährungsprogramms und/oder der Art des wenigstens einen Diäthilfsmittels und/oder der Art und/oder der Anzahl weiterer Diäthilfsmittel bestimmbar ist.

Die wenigstens eine Einrichtung zur Ermittlung des wenigstens einen aktuellen Körperwerts Wₐₖₜ ist vorzugsweise eine Körperwaage und/oder ein Impedanzmessgerät und/oder eine Einrichtung zur Bestimmung der körperlichen Leistungsfähigkeit und/oder ein Pulsmesser und/oder eine Einrichtung zur Bestimmung eines Körpermaßes. Mit diesen vorgenannten Einrichtungen lassen sich repräsentative Körperwerte wie der Body-Mass-Index, das Körpergewicht, die Körperzusammensetzung, insbesondere der Körperfettgehalt oder der Körperwassergehalt, wenigstens ein Körpermaß, insbesondere der Hüftumfang, der Taillenumfang, der Brustumfang, der Oberschenkelumfang oder das Taille/Hüfte-Verhältnis, der PWC-Wert oder der Puls ermitteln.

Besonders bevorzugt ist es, wenn die Einrichtung zur Ermittlung des wenigstens einen aktuellen Körperwerts Wₐₖₜ über eine Datenverbindung mit dem Datenverarbeitungsprogramm bzw. der Datenverarbeitungseinheit verbunden ist. Die Körperwerte können dann automatisch in das Datenverarbeitungsprogramm eingelesen werden.

Die Erfindung wird im Folgenden anhand eines Ausführungsbeispiels weiter erläutert. Dabei wird auf die Zeichnung Bezug genommen, in deren
- FIG 1: eine Datenverarbeitungseinrichtung zur Umsetzung von Körperwerten in Vorgabewerte
- FIG 2: der Verlauf eines Körperwerts
jeweils schematisch dargestellt sind.

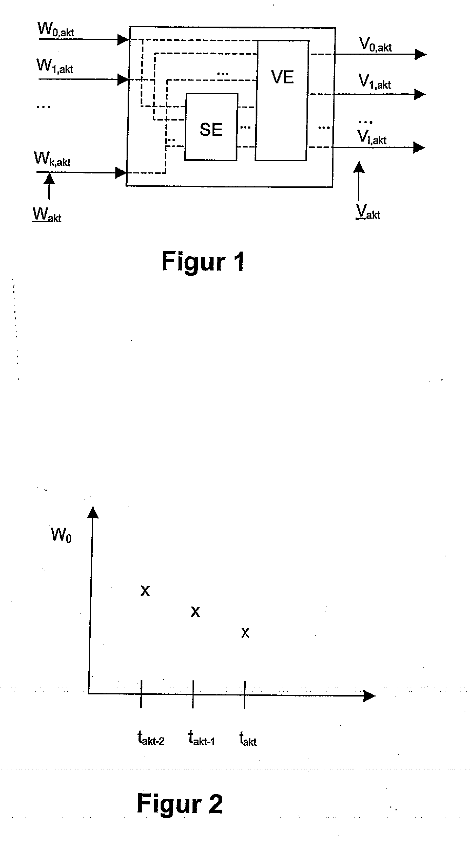

Die Datenverarbeitungseinrichtung in FIG 1 übernimmt die Überwachung der aktuellen Körperwerte Wₐₖₜ und die Steuerung der aktuellen Vorgabewerte Vₐₖₜ zum Erreichen und Sicherstellen eines dauerhaften Diäterfolgs.

Für das Erstellen eines Diätplans mit einem gezielten Ernährungsprogramm, einem Bewegungsprogramm und für die Auswahl und Dosierung der Diäthilfsmittel wird zunächst ein persönlicher Body-Check durchgeführt. Hierfür werden zur Analyse eines Ist-Zustands neben Körpergewicht und Körpergröße der Body-Maß-Index, das Körperfett in % und in kg, die fettfreie Körpermasse in kg, die Körperfettverteilung über das Taille/Hüfte-Verhältnis sowie ein Zielpulsbereich für maximale Fettverbrennung der jeweiligen Person bestimmt.

Die von der Datenverarbeitungseinheit eingelesenen aktuellen Körperwerte Wₐₖₜ können beispielsweise neben Körpergewicht und Körpergröße den Body-Mass-Index, das Körperfett in % und in kg, die fettfreie Körpermasse in kg sowie die Körperfettverteilung über das Taille/Hüfte-Verhältnis enthalten.

Die von der Datenverarbeitungseinheit ermittelten Vorgabewerte Vₐₖₜ können beispielsweise Angaben zur Zusammensetzung der normalen Ernährung, das Bewegungsprogramm, die Dosierung des Diäthilfsmittels, den Sollwert für die Flüssigkeitsaufnahme sowie die Zulässigkeit von alkoholischen Getränken enthalten.

Zur Ermittlung der Vorgabewerte Vₐₖₜ werden in der Datenverarbeitungseinheit bevorzugtermaßen auch die Personenparameter des jeweiligen Benutzers erfasst. Dazu gehören Angaben über Geschlecht, Alter, Größe der jeweiligen Person, körperliche Zustandsinformationen über Rauchen, Schwangerschaft und Stillzeit sowie Informationen über chronische Krankheiten, Allergien sowie Angaben über ständig einzunehmende Arzneimittel.

Die Erfassung und Vorgabe der Werte gliedert sich in drei Abschnitte auf: zunächst werden in der Analysephase (Phase 1) anhand einer Körperanalyse die Zielwerte des Fitnessprogramms festgelegt. Dies umfaßt Werte für die Körperfettmasse, fettfreie Masse, Gewicht sowie den WHR-Wert. Während der Aktivphase (Phase 2) werden die Vorgabewerte Vₐₖₜ so gesteuert, dass die gewünschten Zielwerte erreicht werden. Die Langzeitphase (Phase 3) beginnt nach dem erstmaligen Erreichen der Zielwerte. Hier wird sichergestellt, dass sich ein langfristiger Erfolg einstellt.

Die Datenverarbeitungseinheit gemäß FIG 1 liest an ihren Eingängen die k jeweils aktuellen Körperwerte Wₐₖₜ als Einzelwerte W_{0,akt} bis W_{k,akt} ein. Intern werden diese der Verarbeitungseinheit VE sowie der Speichereinheit SE zugeführt. Die Verarbeitungseinheit VE wertet die Körperwerte W_{0,akt} bis W_{k,akt} zusammen mit den in der Speichereinheit SE abgelegten Werten aus, um die Vorgabewerte Vₐₖₜ individuell auf den Menschentyp und auf den individuellen Änderungsverlauf abstimmen zu können. Das Auswertungsverfahren in der Verarbeitungseinheit VE ist so aktualisierbar, dass jeweils aktuelle Erkenntnisse berücksichtigt werden können. An ihren Ausgängen gibt die Verarbeitungseinheit VE die aktuellen Vorgabewerte Vₐₖₜ als Einzelwerte V_{0,akt} bis V_{1,akt} aus.

In der Speichereinheit SE sind die zuvor ermittelten Werte W_{0,akt-1} bis W_{k,akt-1}, W_{0,akt-2} bis W_{k,akt-2} und W_{0,akt-3} bis W_{k,akt-1}, Personenparameter sowie die Zielwerte abgelegt. Zur Auswertung verwendet die Verarbeitungseinheit VE die aktuellen Körperwerte W_{0,akt} bis W_{k,akt}, sowie die in der Speichereinheit SE abgelegten Werte.

Die konkrete Ermittlung der Vorgabewerte Vₐₖₜ kann basierend auf den aktuellen Körperwerten Wₐₖₜ ggf. unter zusätzlicher Berücksichtigung zuvor ermittelter Werte unter Verwendung analytischer oder numerischer Algorithmen, bevorzugtermaßen auch unter Rückgriff auf Kennfelder erfolgen, wobei bei Rückgriff auf Kennfelder Zwischenpunkte durch Interpolationsverfahren bzw. nicht abgedeckte Punkte durch Extrapolationsverfahren angenährt werden können.

FIG 2 zeigt einen möglichen zeitlichen Verlauf einer Körperwertvariablen W₀, der beispielsweise die Werte des Körpergewichts darstellen könnte, zum aktuellen Auswertungszeitpunkt tₐₖₜ , zum vorhergehenden Auswertungszeitpunkt tₐₖₜ₋₁ und zum vorvorhergehenden Auswertungszeitpunkt tₐₖₜ₋₂.

Während der Analysephase wird ein Diätplan mit Zielwerten für ein Ernährungsprogramm, ein Bewegungsprogramm und für die Auswahl und Dosierung der Diäthilfsmittel erstellt. Hierzu werden zunächst die Personenparameter, also die Angaben über Geschlecht, Alter, Größe sowie körperliche Zustandsinformationen der jeweiligen Person, in der Datenverarbeitungseinheit erfasst. Anschließend werden erstmals die bevorzugtermaßen fünf bis sieben aktuellen Körperwerte Wₐₖₜ ermittelt, ebenfalls in die Datenverarbeitungseinheit eingelesen und ein persönlicher Body-Check durchgeführt. Die aktuellen Körperwerte Wₐₖₜ enthalten Körpergewicht und Körpergröße, den Body-Mass-Index, das Körperfett in % und in kg, die fettfreie Körpermasse in kg sowie die Körperfettverteilung über das Taille/Hüfte-Verhältnis der jeweiligen Person. Während der Analysephase ist der Parameter akt = 0, es werden also erstmals die aktuellen Körperwerte ermittelt.

Zusätzlich kann die Datenverarbeitungseinheit den Zielpulsbereich für Frauen aus (226 - Lebensalter) x 0,6 für eine untere Grenze und (226 - Lebensalter) x 0,7 für eine obere Grenze errechnen. Bei Männern wird der Zielpulsbereich nach der Formel (220 - Lebensalter) x 0,6 bzw. (220 - Lebensalter) x 0,7 berechnet.

Auf Grundlage der eingelesenen Körperwerte, also des Ist-Zustands, definiert die Datenverarbeitungseinheit einen endgültigen Diäterfolg in Form von Zielwerten, die Sollwerte für die Körperwerte Körperfettmasse, fettfreie Masse, Gewicht und den WHR-Wert darstellen. Die Diät für Personen, deren BMI und Körperfettanteil über bzw. deutlich über dem empfohlenen Bereich liegt, wird über einen Zeitraum von wenigstens 15 Wochen festgelegt.

Während der Aktivphase werden jeweils in der 1., 2., 4., 6., 12. und 15. Woche die Körperwerte Körperfettmasse, fettfreie Masse, Gewicht und WHR-Wert gemessen und in die Datenverarbeitungseinheit eingelesen. Hier werden in der Verarbeitungseinheit VE die aktuellen Körperwerte Wₐₖₜ mit den zuletzt ermittelten Körperwerten Wₐₖₜ₋₁ , Wₐₖₜ₋₂ und Wₐₖₜ₋₃ und dem Zielwert abgeglichen und unter Berücksichtigung der eingegebenen Personenparameter die geeignete Dosierung der Diäthilfsmittel als Vorgabewert für den weiteren Diätverlauf ausgegeben. Zudem werden Empfehlungen für eine Anpassung des Bewegungsprogramms und der Ernährung als Vorgabewerte ausgegeben.

Die Datenverarbeitungseinheit stimmt die Vorgabe- und Zielwerte individuell auf den Menschentyp und auf den individuellen Änderungsverlauf ab. Hierzu überprüft sie beispielsweise, wie schnell sich die aktuellen Körperwerte Wₐₖₜ ändern und wie sich das Diäthilfsmittel, das Bewegungsprogramm und das Ernährungsprogramm für die jeweilige Person am besten kombinieren lassen.

Die Ernährung wird so vorgegeben, dass sie eiweissreich ist, also ausreichend viel Fleisch eingenommen wird, und so, daß wenige Kohlenhydrate, jedoch viel Gemüse und Vitamine und hochwertige Fette eingenommen werden. Hierdurch wird auch die Insulinproduktion optimiert.

Als Diäthilfsmittel werden ein diätetisches Lebensmittel in Form eines Shakes 1 bis 2 mal täglich als Mahlzeitenersatz vorgegeben. Zudem wird von der Datenverarbeitungseinheit eine Mindestmenge für die Flüssigkeitsaufnahme abhängig vom aktuellen Status vorgegeben, die täglich 1 bis 3 Portionen eines Basenpulvers (Restorate^{®}) und täglich etwa 1 1 eines basischen Tees (Herbaslim^{®}) beinhaltet.

Das vorgegebene Bewegungsprogramm umfasst zu Beginn (wenige Tage) tägliches Ausdauertraining von 10 min im individuellen Zielpulsbereich. Anschließend wird jedes 4. Training um 2 min gesteigert bis eine tägliche Belastung von 30 min im Zielpulsbereich erreicht wird. Als sportliche Aktivitäten können dabei beispielsweise laufen, schnelles gehen (Walking), schwimmen oder Rad fahren gewählt werden.

Durch die erhöhte Bewegung verstärkt sich der Sauerstoffaufbau im Blut, der Stoffwechsel verschiebt sich in den aeroben Bereich. Hierdurch erhöht sich die Fettverbrennung in den Muskeln. Dieser Effekt wird durch die Einnahme des Diäthilfsmittels noch weiter verstärkt, da die Wirkstoffspeicher im Körper durch die Inhaltsstoffe des Diäthilfsmittels gefüllt sind und so bei erhöhter Bewegung ein intensiverer Muskelaufbau erfolgen kann. Durch die intensivere Muskelarbeit kann wiederum mehr Fett abgebaut werden, was die Gewichtsabnahme wirksam unterstützt.

Die Einnahme des Diäthilfsmittels gewährleistet zudem, dass der Körper während der Diät laufend mit wichtigen Substanzen versorgt wird. Die Einnahme von alkoholischen Getränken kann in der Anfangsphase der Diät beispielsweise als nicht zulässig vorgegeben werden. Bei Diäterfolg ist die Einnahme dann nach und nach wieder zulässig.

Nach Erreichen des gewünschten Diäterfolgs geht die Überwachung durch die Datenverarbeitungseinrichtung in die Kontrollphase über. Hier wird beispielsweise die Einnahme des Diäthilfsmittels schrittweise reduziert. Durch die laufende Kontrolle der Wirksamkeit der Maßnahmen über die in FIG 1 dargestellte Datenverarbeitungseinrichtung entsteht jedoch ein dauerhafter Erfolg, da die Körperwerte Wₐₖₜ auch nach Erreichen des gewünschten Diäterfolgs über einen längeren Zeitraum überwacht werden. Falls die Körperwerte Wₐₖₜ deutlich von den Zielwerten abweichen, greift die Steuerung wieder regulierend ein und verändert die Vorgabewerte Vₐₖₜ für Bewegung, Ernährung und Diäthilfsmittel. Dem so genannten "Jo-Jo-Effekt" wird auf diese Weise wirksam entgegengesteuert.

Ein Beispiel für ein erfindungsgemäßes diätetisches Lebensmittel in Form eines Shakes als Diäthilfsmittel ist im Folgenden angeführt:

**Tabelle 3: FitLine Soja Shake - Cappucino (Zutaten pro 100 g)**

| | | | |
|---|---|---|---|
| Fructose | 50 g | Maltodextrin | 35,0 mg |
| Sojaeiweiß | 28,1 g | Eisen(II)-Lactat | 29,0 mg |
| Sonnenblumenöl | 5,1 g | Hefe, selenreich | 16,4 mg |
| Weizenfaser | 3,2 g | Niacin | 10,7 mg |
| Haferkleie | 3,0 g | Vitamin E | 5,9 mg |
| Kaliumcitrat | 1,98 g | Mangan(II)-Gluconat | 5,2 mg |
| Calciumcitrat | 1,96 g | Kupfer(II)-Gluconat | 4,7 mg |
| Verdickungsmittel: Guakernmehl (E412), Pektin (E 440) | 1,9 g | Pantothenat | 2,3 mg |
| Weizendextrin | 1,5 g | Farbstoff: Beta-Carotin (E 160) | 1,2 mg |
| Instantkaramell | 1,2 g | Vitamin B6 | 1,1 mg |
| Kaliumphosphat | 0,78 g | Vitamin B2 | 0,95 mg |
| Aroma | 0,5 g | Vitamin B1 | 0,7 mg |
| Magnesiumkarbonat | 0,41 g | Vitamin A | 476 µg |
| Papaya-Fruchtpulver | 0,1 g | Folsäure | 120 µg |
| Natriumkarbonat | 50,0 mg | Biotin | 75 µg |
| Zinkgluconat | 45,7 mg | Kaliumjodid | 46 µg |
| Ananas-Fruchtpulver | 45,0 mg | Vitamin D | 3 µg |
| Vitamin C | 35,7 mg | Vitamin B12 | 0,84 µg |
| | | | |
| Isoflavongehalt | 33 mg | | |
| | | | |

**Tabelle 4: FitLine Soja Shake - Cappucino (Durchschnittlicher Gehalt pro 100 g Pulver)**

| | | | |
|---|---|---|---|
| Brennwert | 1520 kJ/ 360 kcal | | |
| | | | |
| Eiweiß | 23 g | | |
| Kohlenhydrate - davon Zucker | 52 g 51 g | | |
| Fett - davon gesättigte Fettsäuren - davon Linolsäure | 6,2 g 1,0 g 3,1 g | | |
| Ballaststoffe | 8,4 g | | |
| | | Vitamin C | 35,7 mg |
| Natrium | 360 mg | Niacin | 10,7 mg |
| Kalium | 1143 mg | Vitamin E | 5,9 mg |
| Calcium | 464 mg | Pantothensäure | 2,3 mg |
| Magnesium | 125 mg | Vitamin B6 | 1,1 mg |
| Phosphor | 357 mg | Vitamin B2 | 0,95 mg |
| Eisen | 9,3 mg | Vitamin B1 | 0,7 mg |
| Zink | 7,3 mg | Vitamin A | 476 µg |
| Jod | 84 µg | Folsäure | 120 µg |
| Kupfer | 0,66 mg | Biotin | 75 µg |
| Mangan | 0,59 mg | Vitamin D | 3 µg |
| Selen | 32,7 µg | Vitamin B12 | 0,84 µg |
| | | | |
| 100 ml des verzehrfertigen Produkts zubereitet als Mahlzeitenersatz (56 g | | | |
| mit 250 ml Wasser) enthalten 287 kJ/69 kcal | | | |
| 100 ml des verzehrfertigen Produkts zubereitet als Tagesration (4 x 42 g | | | |
| mit 200 ml Milch (1,5 % Fett)) enthalten 445 kJ/106 kcal | | | |

### Bezugszeichenliste

- Vₐₖₜ: aktuelle Vorgabewerte
- Wₐₖₜ: aktuelle Körperwerte

- V_{0,akt}: erster Vorgabewert zum aktuellen Auswertungszeitpunkt
- V_{1,akt}: zweiter Vorgabewert zum aktuellen Auswertungszeitpunkt
- V_{1,akt}: letzter Vorgabewert zum aktuellen Auswertungszeitpunkt

- W_{0,akt}: erster Körperwert zum aktuellen Auswertungszeitpunkt
- W_{1,akt}: zweiter Körperwert zum aktuellen Auswertungszeitpunkt
- W_{k,akt}: letzter Körperwert zum aktuellen Auswertungszeitpunkt

- VE: Verarbeitungseinheit
- SE: Speichereinheit

- t: Zeitpunkte
- tₐₖₜ: aktueller Auswertungszeitpunkt
- tₐₖₜ₋₁: vorhergehender Auswertungszeitpunkt
- tₐₖₜ₋₂: vorvorhergehender Auswertungszeitpunkt
- W₀: Körperwertvariable

## Patentansprüche

1. Diätetisches Lebensmittel, beinhaltend wenigstens ein Sojaeiweiß, wenigstens einen Mikrofaser-Ballaststoff, wenigstens einen Kohlenhydrat-Lieferanten, wenigstens einen Fettsäure-Lieferanten, wenigstens ein fettlösliches und wenigstens ein wasserlösliches Vitamin, wenigstens einen Mineralstoff, wenigstens einen Geschmacksstoff und Isoflavone.

2. Diätetisches Lebensmittel nach Anspruch 1, bei dem der Isoflavongehalt zwischen 10 mg/100g diätetisches Lebensmittel und 200 mg/100g diätetisches Lebensmittel, vorzugsweise zwischen 30 mg/100g diätetisches Lebensmittel und 60 mg/100g diätetisches Lebensmittel, insbesondere bei 33 mg/100g diätetisches Lebensmittel, liegt.

3. Diätetisches Lebensmittel nach Anspruch 1 oder Anspruch 2, bei dem das wenigsten eine fettlösliche Vitamin, insbesondere Vitamin A, D und E, in micellierter Form vorliegt.

4. Diätetisches Lebensmittel nach einem der vorangehenden Ansprüche zur Verwendung in einem Verfahren zur Überwachung bzw. Steuerung eines Diäterfolgs während einer Diät, die unter Einnahme wenigstens eines Diäthilfsmittels in einer vorgegebenen Dosierung erfolgt und durch ein vorgegebenes Bewegungsprogramm und/oder ein vorgegebenes Ernährungsprogramm unterstützt wird, bei dem zunächst ein oder mehrere anfängliche Körperwerte W₀ ermittelt werden und dann in vorgegebenen oder beliebigen Zeitabständen wenigstens eine erneute Ermittlung dieser Körperwerte zur Erzielung aktualisierter Vorgabewerte Vₐₖₜ für die Dosierung des wenigstens eines Diäthilfsmittels, eines vorgegebenen Bewegungsprogramms und/oder eines vorgegebenes Ernährungsprogramms stattfindet, unter Berücksichtigung des wenigstens einen aktuellen Körperwertes Wₐₖₜ sowie ggf. unter Berücksichtigung zuvor ermittelter Körperwerte W_{akt-z} (z= 1 .. akt),
wobei zumindest die Dosierung des wenigstens einen Diäthilfsmittels eingestellt wird, bis der mindestens eine aktuelle Körperwert Wₐₖₜ in einem als Diäterfolg definierten Zielbereich liegt,
wobei
k >= 0,
1 >= 0,
Wₓ = [W_{0,x},.. , W_{k,x}],
Vₓ = [V_{0,x},.. , V_{1,x}],
und k bzw. 1 die Anzahl unterschiedlicher Körperwerte bzw. Vorgabewerte zu einem Zeitpunkt tₓ ist.

5. Diätetisches Lebensmittel nach einem der Ansprüche 1 bis 3 zur Verwendung in einem Verfahren mit den Merkmalen gemäß Anspruch 4, bei dem in Abhängigkeit des ermittelten wenigstens einen Vorgabewerts Vₐₖₜ das vorgegebene Bewegungsprogramm und/oder das vorgegebene Ernährungsprogramm angepasst werden bzw. wird bis der gewünschte, endgültige Diäterfolg erreicht ist.

6. Diätetisches Lebensmittel nach einem der Ansprüche 1 bis 3 zur Verwendung in einem Verfahren mit den Merkmalen gemäß Anspruch 4 oder 5, bei dem in Abhängigkeit des ermittelten wenigstens einen aktuellen Vorgabewerts Vₐₖₜ wenigstens ein Diäthilfsmittel ausgetauscht wird und/oder bei dem in Abhängigkeit des ermittelten wenigstens einen aktuellen Vorgabewerts Vₐₖₜ wenigstens ein weiteres Diäthilfsmittel abgesetzt wird.

7. Diätetisches Lebensmittel nach einem der Ansprüche 1 bis 3 zur Verwendung in einem Verfahren mit den Merkmalen gemäß einem der Ansprüche 4 bis 6, bei dem als Körperwert(e) der Body-Mass-Index und/oder das Körpergewicht und/oder die Körperzusammensetzung, insbesondere der Körperfettgehalt und/oder der Körperwassergehalt, und/oder wenigstens ein Körpermaß, insbesondere der Hüftumfang und/oder der Taillenumfang und/oder der Brustumfang und/oder der Oberschenkelumfang und/oder das Taille/Hüfte-Verhältnis, und/oder wenigstens ein Maß für die körperliche Leistungsfähigkeit, insbesondere der PWC-Wert und/oder der Puls, ermittelt werden bzw. wird.

8. Diätetisches Lebensmittel nach einem der Ansprüche 1 bis 3 zur Verwendung in einem Verfahren mit den Merkmalen gemäß einem der Ansprüche 4 bis 7, bei dem der ermittelte wenigstens eine aktuelle Körperwert Wₐₖₜ, und/oder wenigstens eine dem Körperwert zugeordnete Zeitangabe tₐₖₜ, insbesondere ein Datum oder eine Zeitspanne zu einem vorhergehend ermittelten wenigstens einen aktuellen Körperwert W_{akt-z} (z= 1 .. akt), und/oder wenigstens ein Personenparameter, insbesondere die Größe und/oder das Geschlecht und/oder das Alter und/oder wenigstens ein körperlicher Zustand und/oder wenigstens eine medizinische Information, an eine Datenverarbeitungseinheit übertragen wird oder in eine Datenverarbeitungseinheit eingegeben wird und die Datenverarbeitungseinheit die Dosierung des wenigstens einen Diäthilfsmittels und/oder das Bewegungsprogramm und/oder das Ernährungsprogramm in Abhängigkeit des wenigstens einen Körperwerts und/oder der wenigstens einen dem Körperwert zugeordneten Zeitangabe und/oder des wenigstens einen Personenparameters ermittelt.

9. Diätetisches Lebensmittel nach einem der Ansprüche 1 bis 3 zur Verwendung in einem Verfahren mit den Merkmalen gemäß einem der Ansprüche 4 bis 8, bei dem ein Datenverarbeitungsprogramm den wenigstens einen aktuellen Körperwert Wₐₖₜ mit einem optimalen Körperwert und/oder einem zu einem vorgehenden Zeitpunkt ermittelten Körperwert W_{akt-z} (z= 1 .. akt) vergleicht und das Datenverarbeitungsprogramm die Dosierung des wenigstens einen Diäthilfsmittels und/oder das Bewegungsprogramm und/oder das Ernährungsprogramm in Abhängigkeit des wenigstens einen aktuellen Körperwerts Wₐₖₜ und/oder der wenigstens einen dem Körperwert zugeordneten Zeitangabe tₐₖₜ und/oder des wenigstens einen Personenparameters ermittelt.

10. Diätetisches Lebensmittel nach einem der Ansprüche 1 bis 3 zur Verwendung in einem Verfahren mit den Merkmalen gemäß einem der Ansprüche 4 bis 9, bei dem Daten mehrerer Personen, insbesondere der jeweilige wenigstens eine aktuelle Körperwert Wₐₖₜ und/oder die wenigstens eine dem Körperwert zugeordnete Zeitangabe tₐₖₜ und/oder der wenigstens eine Personenparameter, in eine zentrale Datenverarbeitungseinheit eingegeben oder an die zentrale Datenverarbeitungseinheit übertragen werden, insbesondere über eine Datenverbindung, vorzugsweise über das Internet, bei dem das Datenverarbeitungsprogramm personenspezifisch die Dosierung des wenigstens einen Diäthilfsmittels und/oder das Bewegungsprogramm und/oder das Ernährungsprogramm ermittelt und an die jeweilige Person ausgibt oder ausliest und überträgt.

11. Diätetisches Lebensmittel nach einem der Ansprüche 1 bis 3 zur Verwendung in einem Verfahren mit den Merkmalen gemäß einem der Ansprüche 4 bis 10, bei dem als Diäthilfsmittel wenigstens ein Mittel zur Entsäuerung, insbesondere ein basischer Tee und/oder ein Basenpulver, und/oder ein diätetisches Lebensmittel als Mahlzeitenersatz und/oder ein Nahrungsergänzungsmittel und/oder ein Wirkstoffpräparat eingenommen wird.

12. Diätetisches Lebensmittel nach einem der Ansprüche 1 bis 3 als Bestandteil eines Systems bzw. einer Anordnung zur Überwachung bzw. Steuerung eines Diäterfolgs, während einer Diät, die unter Einnahme wenigstens eines Diäthilfsmittels in einer vorgegebenen Dosierung erfolgt und durch ein vorgegebenes Bewegungsprogramm und/oder ein vorgegebenes Ernährungsprogramm unterstützt wird, insbesondere zur Durchführung des Verfahrens nach einem oder mehreren der vorhergehenden Ansprüche, umfassend wenigstens eine Einrichtung zur Ermittlung wenigstens eines aktuellen Körperwerts Wₐₖₜ, eine vorgegebene Menge wenigstens eines Diäthilfsmittels und wenigstens ein Datenverarbeitungsprogramm zur Auswertung des wenigstens einen aktuellen Körperwerts Wₐₖₜ, wobei mit Hilfe des Datenverarbeitungsprogramms in Abhängigkeit des ermittelten wenigstens einen Körperwerts zumindest eine optimale Dosierung des wenigstens einen Diäthilfsmittels bestimmbar ist.

13. Diätetisches Lebensmittel nach einem der Ansprüche 1 bis 3 als Bestandteil eines Systems bzw. einer Anordnung mit den Merkmalen gemäß Anspruch 12,
wobei mit Hilfe des Datenverarbeitungsprogramms in Abhängigkeit des ermittelten wenigstens einen aktuellen Körperwerts Wₐₖₜ das Bewegungsprogramm und/oder das vorgegebene Ernährungsprogramm und/oder die Art des wenigstens einen Diäthilfsmittels und/oder die Art und/oder die Anzahl weiterer Diäthilfsmittel bestimmbar ist sowie in Abhängigkeit eines Diätverlaufs eine Anpassung oder Abänderung der Dosierung des wenigstens einen Diäthilfsmittels und/oder des Bewegungsprogramms und/oder des vorgegebenen Ernährungsprogramms und/oder der Art des wenigstens einen Diäthilfsmittels und/oder der Art und/oder der Anzahl weiterer Diäthilfsmittel bestimmbar ist.

14. Diätetisches Lebensmittel nach einem der Ansprüche 1 bis 3 als Bestandteil eines Systems bzw. einer Anordnung mit den Merkmalen gemäß Anspruch 12 oder 13,
wobei die wenigstens eine Einrichtung zur Ermittlung des wenigstens einen aktuellen Körperwerts Wₐₖₜ eine Körperwaage und/oder ein Impedanzmessgerät und/oder eine Einrichtung zur Bestimmung der körperlichen Leistungsfähigkeit und/oder ein Pulsmesser und/oder eine Einrichtung zur Bestimmung eines Körpermaßes ist.

15. Diätetisches Lebensmittel nach einem der Ansprüche 1 bis 3 als Bestandteil eines Systems bzw. einer Anordnung mit den Merkmalen gemäß einem der Ansprüche 12 bis 14,
wobei die Einrichtung zur Ermittlung des wenigstens einen aktuellen Körperwerts Wₐₖₜ über eine Datenverbindung mit dem Datenverarbeitungsprogramm verbunden ist.
